# EUROPÄISCHE PATENTSCHRIFT

(11) **EP 0 925 076 B1**
(45) Veröffentlichungstag und Bekanntmachung des Hinweises auf die Patenterteilung: **20.02.2002**
(21) Anmeldenummer: 97938672.9
(22) Anmeldetag: 10.09.1997
(51) Int. Cl.: A61L 27/00, A61K 9/14

(54) **VERFAHREN ZUR HERSTELLUNG EINES IMPLANTATES, BESTEHEND AUS EINEM RESORBIERBAREN TRÄGERMATERIAL, EINE MEDIZINISCHE WIRKSUBSTANZ WIE EIN PHARMAZEUTIKA, ANTIBIOTIKA, CYTOSTATIKA ODER HORMON ENTHALTEND**
METHOD TO PRODUCE AN IMPLANT CONSISTING OF AN ABSORBABLE CARRIER MATERIAL CONTAINING A MEDICAL ACTIVE SUBSTANCE SUCH AS A PHARMACEUTICAL SUBSTANCE, AN ANTIBIOTIC, A CYTOSTATIC, OR A HORMONE
PROCEDE DE PRODUCTION D'UN IMPLANT COMPOSE D'UN MATERIAU SUPPORT RESORBABLE RENFERMANT UNE PHARMACEUTICE, UN ANTIBIOTIQUE, UN CYTOSTATIQUE OU UNE HORMONE

(30) Priorität: 10.09.1996 AT 160696
(43) Veröffentlichungstag der Anmeldung: 30.06.1999
(73) Patentinhaber: Mediphore-Biotechnologie AG, 1010 Wien (AT)
(72) Erfinder: Winkler, Heinz, 1232 Wien (AT)
(74) Vertreter: Wildhack, Helmut, Dr. Dipl.-Ing.
(86) Internationale Anmeldenummer: AT9700198
(87) Internationale Veröffentlichungsnummer: WO9810802

(56) Entgegenhaltungen:
- EP-A- 0 419 275
- WO-A-86/07265
- WO-A-89/11880

## Beschreibung

Die Erfindung betrifft ein Verfahren zur Herstellung eines Implantates, bestehend aus einem resorbierbaren Trägermaterial eine medizinische Wirksubstanz, wie ein Pharmazeutika, Antibiotika, Cytostatika, oder Hormon enthaltend.

Bei orthopädischen Operationen treten häufig Infektionen im Knochengewebe (Osteomyelitis) auf, die durch Antibiotikagaben bekämpft werden müssen. Intravenös oder oral verabreichte Antibiotikagaben bleiben häufig, auch bei günstiger Empfindlichkeit des Erregers, ohne anhaltenden Heilungseffekt. Grund dafür ist die schlechte Erreichbarkeit der infizierten Abschnitte bei schlechten Durchblutungsverhältnissen, infolge Narbenbildung bzw. Sklerosierung sowie für Antibiotika schwer durchdringliche, membranartige Strukturen um die Bakterienkolonien. Zur Bekämpfung der Infektionen mittels intravenös oder oral verabreichter Antibiotika sind aus diesem Grunde Langzeitgaben in hohen Dosierungen unvermeidlich. Einer Hochdosierung sind jedoch durch das Auftreten systemischer Nebenwirkungen Grenzen gesetzt.

Bei Auftreten von Infektionen im Knochengewebe ist daher häufig eine zusätzliche chirurgische Intervention erforderlich, wobei alle besiedelten Gewebeanteile entfernt werden. Nach chirurgischem Debridement sind Knochendefekte eine unvermeidliche Folge. Zu deren Auffüllung werden Knochentransplantate verwendet, wobei zahlreiche Probleme auftreten. So sind Knochentransplantate primär avital und daher ein idealer Nährboden für eine neuerliche Keimbesiedelung. In der Regel werden diese Knochentransplantate daher erst in einem weiteren Schritt nach Beherrschung der Infektion eingebracht. Andernfalls besteht die Gefahr der Sequestrierung und Persistenz der Infektion.

Man hat daher bereits vorgeschlagen, Antibiotika lokal im Bereich der Infektion zu applizieren, jedoch konnten hier nur Teilerfolge erzielt werden.

Lokale Instillationen sind entweder zu kurz wirksam oder erfordern das Anlegen von aufwendigen Zuleitungssystemen. In klinischer Verwendung stehen derzeit praktisch ausschließlich Antibiotikaträger in Form von Polymethylmetacrylat, die mit Gentamycin inkubiert sind. Andere Antibiotika sind mit einem solchen Träger kaum kombinierbar, sodaß die Anwendung schon aus diesem Grunde beschränkt ist. Die erreichten Gewebespiegel liegen zwar höher als bei einer intravenösen oder oralen Antibiotikagabe, sind jedoch zumeist trotzdem nicht ausreichend zur Elimination resistenter Keime. Ein wesentlicher Nachteil derartiger Antibiotikaträger ist es, daß sie nach einigen Tagen Liegezeit wieder entfernt werden müssen. Um diesen Nachteil auszuschalten, wurden in der jüngsten Vergangenheit resorbierbare Implantate entwickelt, die aus mit Gentamycin getränkten Kollagenschwämmen tierischer Herkunft bestehen. Für andere Antibiotika ist dieses Verfahren bisher nicht anwendbar und derartige Implantate verfügen gleichfalls nur über eine Wirkdauer von einigen Tagen.

Bei anderen Behandlungsfällen soll nicht oder nicht nur eine lokale Applikation von Antibiotika durch Implantate erfolgen, sondern auch anderer medizinischer Wirksubstanzen. So besitzen Transplantate, die zur Auffüllung von Knochendefekten Verwendung finden, in der Regel keine osteoinduktive Potenz, d.h. es erfolgt keine Anregung zur Knochenneubildung. Eine Regeneration von Defekten ist daher nicht induzierbar. Sie wirken lediglich als Platzhalter, entlang deren Strukturen sich neues körpereigenes Gewebe formieren soll. Der Zusatz von entsprechenden Faktoren, die die Knochenregeneration anregen, erscheint daher nützlich. Von diesen sind bereits zahlreiche identifiziert und teilweise sogar auf gentechnologischer Basis herstellbar. In der klinischen Anwendung bestehen jedoch weiterhin Probleme, da sie nicht in genügend hoher Konzentration und gleichzeitig genügend lange im erwünschten Wirkort appliziert werden können.

In der Chirurgie von malignen Tumoren ist in bestimmten Fällen eine hohe Lokalkonzentration von Cytostatika erwünscht. In derartigen Fällen ist die Vermeidung systemischer Wirkungen besonders erstrebenswert, da daraus resultierende Organschädigungen mitunter lebensbedrohliche Ausmaße annehmen können.

Auch bei anderen Wirksubstanzen (Hormone, Pharmazeutika etc.) ist mitunter deren lokal begrenzte bzw. lang und kontinuierlich anhaltende Wirkung erstrebenswert. Durch Implantation geeigneter Träger mit der gewünschten Wirksubstanz können diese Ziele erreicht werden.

Aus der US 4,882,149 A ist ein Trägermaterial bekanntgeworden, bei welchem nach der Waschbehandlung eine Lufttrocknung bei 100°C erfolgt. Eine derart hohe Temperatur schädigt die molekularen Strukturen des Trägermateriales und bewirkt ein Schrumpeln desselben, wodurch die Hohlräume des Trägermateriales, die ja für die Fähigkeit der Menge der aufzunehmenden Wirksubstanz bestimmend sind, so verkleinert werden, daß nur eine geringe Menge dieser Wirksubstanz im Trägermaterial speicherbar ist. Wird Knochenmaterial als Trägermaterial verwendet, so entsteht bei einer derartigen Behandlung Leim, der überhaupt nicht mehr aufnahmefähig für Wirksubstanzen ist.

Aus der EP 419 275 A1 ist ein demineralisierter Knochenpuder mit einem Medikament als weiteren Bestandteil bekannt geworden.

Die WO 86/07265 offenbart ein Implantat, das aus natürlichen Knochenmaterialien und adsorbierten physiologischen Substanzen, wie beispielsweise einem Antibiotikum, besteht.

Die vorliegende Erfindung hat sich zur Aufgabe gestellt, ein Verfahren zur Herstellung eines Implantates vorzuschlagen, welches Implantat aus einem Trägermaterial besteht, welches vorzugsweise resorbierbar ist und mit dem eine lokale Applikation von medizinischen Wirksubstanzen verschiedenster Art in veränderlicher Dosierung über variierbare Zeiträume ermöglicht wird. Zur Lösung dieser Aufgabe wird vorgeschlagen, daß ein das Trägermaterial bildender, organischer Stoff, insbesondere ein biologisches Gewebe menschlichen, tierischen oder pflanzlichen Ursprungs, beispielsweise Knochen, Sehnen, Muskeln od.dgl., zerteilt, gereinigt und einer Gefriertrocknung unterzogen wird, worauf dieses Trägermaterial mit einer die Wirksubstanzen enthaltenden Lösung inkubiert wird. Durch eine solche Gefriertrocknung vor der Inkubation wird der das Trägermaterial bildende organische Stoff so präpariert, daß er einerseits so trocken wie möglich ist und daher eine sehr große Menge der Wirksubstanz aufzunehmen vermag, andererseits nicht geschädigt wird und seine Strukturen sowohl im molekularen als auch im mikroskopischen und makroskopischen Bereich vollständig erhalten bleiben. Ein nach dem erfindungsgemäßen Verfahren hergestelltes Implantat weist somit den Vorteil auf, daß bei Inkubation mit der die Wirksubstanz enthaltenden Lösung eine sehr gute Anreicherung entsprechend ihres Konzentrationsgradienten sichergestellt ist, wobei eine vollständige Rehydration des gefriergetrockneten Stoffes erfolgt und die Wirksubstanz in entsprechend hoher Dosis im organischen Stoff abgelagert, adsorbiert und gegebenenfalls molekular eingebaut wird. Durch Wahl des Trägermaterials, der Partikelgröße, der Konzentration der die Wirksubstanz enthaltenden Lösung und der Inkubationszeit können die Wirkungsintensität und die Wirkungsdauer der Wirksubstanz gesteuert und somit an die jeweiligen Erfordernisse angepaßt werden.

Ein Implantat nach erfolgter Inkubation einer Gefriertrocknung zu unterziehen, ist bereits bekannt. Die Gefriertrocknung dient in diesem Fall der Haltbarmachung des Implantates und hat, da die Inkubation des Trägermaterials bereits vor der Gefriertrocknung stattfindet, auf das Verhalten des Trägermaterials bei der Inkubation keinen Einfluß.

Es liegt auf der Hand, daß nach dem Zerteilen unerwünschte Teile des Trägermaterials, also beispielsweise Gewebeteile zwischen den eigentlichen Trägerstrukturen (Knochentrabekel) entfernt werden sollten, sodaß sich durch vergrößerte Kontaktflächen eine bessere Perfusionsfähigkeit mit vermehrter Aufnahme der die Wirksubstanzen enthaltenden Lösung ergibt.

Die erforderliche Reinigung des organischen Stoffes erfolgt gemäß einem bevorzugten Verfahrensschritt mittels einer, vorzugsweise auf eine Temperatur zwischen 40°C und 60°C, erwärmten Spülflüssigkeit, die beispielsweise durch Ultraschall und/oder durch Schütteln des aufnehmenden Gefäßes, bewegt wird.

Zweckmäßig wird der organische Stoff vor der Gefriertrocknung einer Entfettung durch Behandlung mit einer fettlösenden Substanz, beispielsweise mit Äther, unterzogen. Dadurch wird einerseits die Benetzbarkeit der Oberfläche des organischen Stoffes durch die die Wirksubstanz enthaltende Lösung vergrößert und andererseits auch eine verstärkte Aufnahmefähigkeit des organischen Stoffes für fettige bzw. ölige Lösungen geschaffen, sodaß auch solche fettige bzw. ölige Lösungen in hohem Ausmaß im Trägermaterial gespeichert werden können. Im Anschluß kann der organische Stoff mit Alkohol behandelt und anschließend in keimfreiem Wasser gespült werden.

Gemäß einem weiteren bevorzugten Verfahrensschritt wird der organische Stoff vor der Gefriertrocknung einer Behandlung zur Vergrößerung seiner Oberfläche, beispielsweise einer Mikrofrakturierung mittels Ultraschall unterworfen, wodurch die zur Adsorption zur Verfügung stehende Oberfläche des organischen Stoffes weiter vergrößert wird.

Schließlich kann der organische Stoff nach der Gefriertrocknung einer ionisierenden Strahlung ausgesetzt werden, durch welche im Trägermaterial molekulare Veränderungen induziert werden, die eine erhöhte Bindungsfähigkeit mit der Wirksubstanz bewirken.

Eine noch höhere Speicherfähigkeit des gefriergetrockneten Stoffes für die Wirksubstanzen wird dann erzielt, wenn die Inkubation des gefriergetrockneten Stoffes mit einer die Wirksubstanz enthaltenden Lösung im Vakuum erfolgt, da dann die Lösung samt den gelösten Wirksubstanzen unbehindert bis in die tiefsten Strukturen des Trägermaterials vordringen kann.

Es hat sich gezeigt, daß günstige Ergebnisse dann erzielt werden, wenn die Gefriertrocknung des organischen Stoffes bis zu einem Restfeuchtegehalt unter 10%, vorzugsweise von maximal 5% erfolgt.

Eine besonders schonende Behandlung des Trägermaterials ergibt sich bei Anwendung einer Gefriertrocknung bei einer Temperatur zwischen -20°C und -40°C, vorzugsweise von -30°C.

Im folgenden wird die Anwendung des erfindungsgemäßen Verfahrens anhand eines Beispieles erörtert.

Von dem das Trägermaterial bildenden Knochengewebe werden die Weichteile, Knorpel und sklerosierter Knochen entfernt und das Knochengewebe wird in Standardform und -größen zugeschnitten. Gegebenenfalls kann eine weitere Zerkleinerung in einer Knochenmühle erfolgen.

Das so präparierte Knochengewebe wird im folgenden einer Grundreinigung mittels Ultraschall unterzogen. Hiebei wird das Knochengewebe in die Wanne eines Ultraschall-Reinigungsgerätes, welche auf eine Temperatur von mindestens 40°C und maximal 60°C erwärmtes, ultrafiltriertes Wasser enthält, gefüllt und dort 15 Minuten lang mit einer Frequenz von etwa 35 Khz beschallt. Sodann wird das Wasser gewechselt und der Reinigungsvorgang weitere 15 Minuten lang wiederholt. Durch die Ultraschallbehandlung erfolgt auch eine Mikro-Fragmentierung, wodurch die Oberfläche des Trägermaterials vergrößert und dadurch die Adsorption der die Wirksubstanzen enthaltenden Lösung verbessert wird.

Anschließend wird das Knochengewebe in einem mit Spülwasser gefüllten Behälter einer Schüttelmaschine etwa zwei Stunden lang mit einer Frequenz zwischen 50 und 100 Hz geschüttelt, worauf das Spülwasser erneuert und der Schüttelvorgang zumindest einmal wiederholt wird. Die Gesamtdauer dieser Schüttelreinigung beträgt zwischen vier und acht Stunden.

Anschließend gelangt das Knochengewebe in einen mit Äther gefüllten Behälter, der mit einer niedrigen Frequenz von etwa 60 Hz mindestens drei Stunden lang geschüttelt wird. Dadurch erfolgt eine Entfettung des Knochengewebes. Falls diese nach einem Arbeitsvorgang nicht ausreichend erscheint, wird der Äther gewechselt und der Arbeitsvorgang wiederholt.

Anschließend wird zur Elution des Äthers das Knochengewebe in Schüttelbädern behandelt, die zunächst 70%, dann 50% und letztlich 30% Alkohol und anschließend keimfreies Wasser enthalten. Die Dauer jedes Arbeitsvorganges beträgt etwa ein bis zwei Stunden.

Das so präparierte Knochengewebe wird nun einer Gefriertrocknung in geeigneten Behältern bei einer Temperatur von etwa -30°C unterzogen, bis es eine Restfeuchte von weniger als 5% aufweist (Haupttrocknung: Druck 0,370 mbar, 0°, Delta t minus 10°, 24 h Nachtrocknung: 30 min.) und hierauf in einem evakuierten, luftdicht verschlossenen Behälter gelagert.

Nach einer ionisierenden Bestrahlung des Knochengewebes mit einer Dosis zwischen 20 und 30kgy wird eine wässerige oder ölige Lösung der gewünschten medizinischen Wirksubstanz, beispielsweise eines Antibiotikums, hergestellt und mit dem so vorbereiteten, unter Vakuum stehenden Knochengewebe inkubiert. Durch Variation der Konzentration der Lösung, der Dauer der Inkubation und der Art und Partikelgröße des Trägermateriales kann die Menge der Wirksubstanz im Trägermaterial gesteuert werden. Daraus ergeben sich bei der Anwendung definierte und reproduzierbare Spiegel im zu behandelnden Gewebe bzw. im Serum des Empfängers.

Die Implantation kann unmittelbar anschließend erfolgen, es kann aber auch das so hergestellte Implantat lyophilisiert werden, wobei nur die Feuchtigkeit entzogen wird, die Wirksubstanz aber im Gewebe verbleibt. Dadurch wird eine nahezu unbeschränkte Haltbarkeit und Lagerungsfähigkeit des Implantates gewährleistet. Die Aktivierung erfolgt in einem solchen Fall erst unmittelbar vor Verwendung durch Zufuhr von Wasser oder erst, sobald nach Implantation eine Rehydrierung durch körpereigene Säfte erfolgt. Diese ist abhängig von Durchmesser und Dichte des gewählten Träger-Gewebes und kann daher durch entsprechende Auswahl des Trägers über variabel große Zeiträume aufrechterhalten werden.

## Patentansprüche

1. Verfahren zur Herstellung eines Implantates, bestehend aus eine resorbierbaren Trägermaterial, eine medizinische Wirksubstanz, wie ein Pharmazeutika, Antibiotika, Cytostatika, oder Hormon enthaltend **dadurch gekennzeichnet, daß** ein das Trägermaterial bildender, organischer Stoff, insbesondere ein biologisches Gewebe menschlichen, tierischen oder pflanzlichen Ursprungs, beispielsweise Knochen, Sehnen, Muskeln, zerteilt, gereinigt und einer Gefriertrocknung unterzogen wird, worauf dieses Trägermaterial mit einer, die Wirksubstanz enthaltenden, Lösung inkubiert wird.

2. Verfahren nach Anspruch 1, **dadurch gekennzeichnet, daß** die Reingiung des organischen Stoffes mittels einer, vorzugsweise auf eine Temperatur zwischen 40°C und 60°C erwärmten Spülflüssigkeit erfolgt, die, beispielsweise durch Ultraschall und/oder durch eine Schüttelbewegung des das Wasser aufnehmenden Gefäßes, bewegt wird.

3. Verfahren nach Anspruch 1, **dadurch gekennzeichnet, daß** der organische Stoff vor der Gefriertrocknung einer Entfettung durch Behandlung mit einer fettlösenden Substanz, beispielsweise mit Äther, unterzogen wird.

4. Verfahren nach Anspruch 1 und 3, **dadurch gekennzeichnet, daß** der organische Stoff nach erfolgter Behandlung mit einer fettlösenden Substanz mit Alkohol behandelt und anschließend in keimfreiem Wasser gespült wird.

5. Verfahren nach Anspruch 1, **dadurch gekennzeichnet, daß** der organische Stoff einer Behandlung zur Vergrößerung seiner Oberfläche, beispielsweise mittels Ultraschall, unterworfen wird.

6. Verfahren nach Anspruch 1, **dadurch gekennzeichnet, daß** der organische Stoff nach der Gefriertrocknung einer ionisierenden Strahlung ausgesetzt wird.

7. Verfahren nach Anspruch 1, **dadurch gekennzeichnet, daß** die Inkubation des gefriergetrockneten Stoffes mit einer die Wirksubstanz enthaltenden Lösung im Vakuum erfolgt.

8. Verfahren nach Anspruch 1, **dadurch gekennzeichnet, daß** die Gefriertrocknung des Trägermateriales bis zu einem Restfeuchtegehalt unter 10%, vorzugsweise von maximal 5%, erfolgt.

9. Verfahren nach Anspruch 1, **dadurch gekennzeichnet, daß** die Gefriertrocknung des Trägermateriales bei einer Temperatur zwischen -20°C und -40°C, vorzugsweise von -30°C erfolgt.

## Claims

1. A method of manufacturing an implant consisting of an absorbable carrier material containing a medical active substance, such as a pharmaceutical substance, an antibiotic. a cytostatic or a hormone, **characterised in that** an organic material forming the carrier material, in particular a biological tissue of human, animal or vegetable origin, for example bone, tendons or muscles is divided, cleaned and subjected to freeze-drying. after which this carrier material is incubated with a solution containing the active substance.

2. A method according to Claim 1, **characterised in that** the organic material is cleaned by means of a rinsing fluid, preferably heated to a temperature between 40°C and 60°C, which is moved for example by ultrasound or by agitating the vessel receiving the water.

3. A method according to Claim 1. **characterised in that**, prior to freeze-drying, the organic material is subjected to fat removal by treatment with a fat-dissolving substance, for example with ether.

4. A method according to Claim 1 and 3, **characterised in that**, following treatment with a fat-dissolving substance, the organic material is treated with alcohol and then rinsed in sterile water.

5. A method according to Claim 1, **characterised in that** the organic material is subjected to a treatment to increase its surface area, for example by means of ultrasound.

6. A method according to Claim 1, **characterised in that**, after freeze-drying, the organic material is exposed to ionising radiation.

7. A method according to Claim 1, **characterised in that** incubation of the freeze-dried substance with a solution containing the active substance takes place in a vacuum.

8. A method according to Claim 1, **characterised in that** the carrier material is freeze-dried to give a residual moisture content of less than 10%, preferably maximally 5%.

9. A method according to Claim 1, **characterised in that** freeze-drying of the carrier material takes place at a temperature of between -20 °C and -40 °C, preferably of -30 °C.

## Revendications

1. Procédé de production d'un implant composé d'un matériau support résorbable renfermant une substance médicalement active, telle qu'un produit pharmaceutique, un antibiotique, un cytostatique ou une hormone, **caractérisé en ce qu'**une substance organique constituant le matériau support, en particulier un tissu biologique d'origine humaine, animale ou végétale, en particulier des os, des tendons, des muscles, est découpée, nettoyée et lyophilisée, avant d'être incubée en présence d'une solution contenant la substance médicalement active.

2. Procédé selon la revendication 1, **caractérisé en ce que** le nettoyage de la substance organique s'effectue au moyen d'un liquide de rinçage chauffé de préférence à une température comprise entre 40°C et 60°C et agité par exemple par des ultrasons et/ou par agitation du récipient contenant l'eau.

3. Procédé selon la revendication 1, **caractérisé en ce qu'**avant le traitement de lyophilisation, la substance organique est soumise à un traitement de dégraissage au moyen d'une substance dissolvant la graisse, par exemple de l'éther.

4. Procédé selon les revendications 1 et 3, **caractérisé en ce qu'**après le traitement de dégraissage au moyen d'une substance dissolvant la graisse, la substance organique est traitée à l'alcool avant d'être rincée dans une eau stérile.

5. Procédé selon la revendication 1, **caractérisé en ce que** la substance organique est soumise à un traitement d'accroissement de sa surface, par exemple au moyen d'ultrasons.

6. Procédé selon la revendication 1, **caractérisé en ce que** la substance organique est exposée à un rayonnement ionisant après lyophilisation.

7. Procédé selon la revendication 1, **caractérisé en ce que** l'incubation de la substance lyophilisée en présence d'une solution contenant la substance médicalement active s'effectue sous vide.

8. Procédé selon la revendication 1, **caractérisé en ce que** la lyophilisation du matériau support se prolonge jusqu'à l'obtention d'une humidité résiduelle inférieure à 10 %, de préférence inférieure ou égale à 5 %.

9. Procédé selon la revendication 1, **caractérisé en ce que** la lyophilisation du matériau support s'effectue à une température comprise entre -20°C et -40°C, de préférence -30°C.
